# EUROPEAN PATENT SPECIFICATION

(11) **EP 2 558 850 B1**
(45) Date of publication and mention of the grant of the patent: **08.03.2017**
(21) Application number: 11724438.4
(22) Date of filing: 15.04.2011
(51) Int. Cl.: G01N 27/62, G06K 9/00, G06F 19/00

(54) **ION MOBILITY METHOD AND APPARATUS FOR IDENTIFYING A SAMPLE COMPOUND**
IONENMOBILITÄTSVERFAHREN UND VORRICHTUNG ZUR IDENTIFIKATION EINER PROBENVERBINDUNG
MÉTHODE UTILISANT LA MOBILITÉ IONIQUE ET DISPOSITIF D'IDENTIFICATION D'UN COMPOSÉ ÉCHANTILLON

(30) Priority: 20.05.2010 GB 201008414; 26.04.2010 US 327843 P; 15.04.2010 GB 201006312
(43) Date of publication of application: 20.02.2013
(73) Proprietor: Micromass UK Limited, Wilmslow SK9 4AX (GB)
(72) Inventor: CAMPUZANO, Iain David Grant, Westlake Village, CA 91361-1725 (US); DEAR, Gordon, Brentford Middlesex TW8 9GS (GB); BEAUMONT, Claire, Brentford Middlesex TW8 9GS (GB); ROBERTS, Andrew, Brentford Middlesex TW8 9GS (GB); MURIEDAS, Jordi Munos, Brentford Middlesex TW8 9GS (GB)
(74) Representative: Harding, Andrew Philip
(86) International application number: PCT/GB2011/050756
(87) International publication number: WO 2011/128703

(56) References cited:
- WO-A1-2006/040622
- KNAPMAN T W ET AL: "Considerations in experimental and theoretical collision cross-section measurements of small molecules using travelling wave ion mobility spectrometry-mass spectrometry", INTERNATIONAL JOURNAL OF MASS SPECTROMETRY, ELSEVIER SCIENCE PUBLISHERS, AMSTERDAM, NL, vol. 298, no. 1-3, 4 October 2009 (2009-10-04), pages 17-23, XP027492678, ISSN: 1387-3806, DOI: DOI:10.1016/J.IJMS.2009.09.011 [retrieved on 2009-10-04]
- TAO ET AL: "A Collision Cross-Section Database of Singly-Charged Peptide Ions", JOURNAL OF THE AMERICAN SOCIETY FOR MASS SPECTROMETRY, ELSEVIER SCIENCE INC, US, vol. 18, no. 7, 26 June 2007 (2007-06-26), pages 1232-1238, XP022131654, ISSN: 1044-0305, DOI: DOI:10.1016/J.JASMS.2007.04.003

## Description

This invention relates generally to a method and apparatus for identifying a sample compound. More specifically, although not exclusively, this invention relates to such a method for differentiating isomeric or isobaric compounds.

As an analytical technique, mass spectrometry (MS) has limited capabilities for separating isomeric or isobaric species or for providing structural conformational information. Some structural information can be gained through tandem MS (MS/MS) techniques. There are classes of substituted molecules, such as hydroxylated metabolites which produce identical MS/MS spectra. This is problematic if the exact position of the hydroxylation is required. Ion mobility (IM) has the ability to separate isomeric or isobaric species, such as hydroxylated metabolites, rapidly (msec) based on differences in their collision cross-section (Ω; physical size, and shape) in the gas-phase, thus providing specific information on ionic configuration and therefore, the position of the hydroxyl moiety. The combination of MS with IM provides an extremely powerful analytical tool.

In recent years, the ability to measure a protein or protein/protein complex's collision cross-section (Ω) value has proven to be a very powerful feature and has significantly added to the advance of structural biology. This however, cannot be said about small molecule and metabolite identification. There has been very little addition to the literature in this area of research. However, recent research has begun to investigate, successfully, the separative powers of travelling wave ion mobility for small ruthenium anticancer structural isomers or isobars.

The following prior art is known:
- KNAPMAN T W ET AL: "Considerations in experimental and theoretical collision cross-section measurements of small molecules using travelling wave ion mobility spectrometry-mass spectrometry", INTERNATIONAL JOURNAL OF MASS SPECTROMETRY, ELSEVIER SCIENCE PUBLISHERS, AMSTERDAM, NL, vol. 289, no. 1-3 4 October 2009 (2009-10-04), pages 17-23, XP027492678
- TAO ET AL: "A Collision Cross-Section Database of Singly-Charged Peptide Ions" JOURNAL OF THE AMERICAN SOCIETY FOR MASS' SPECTROMETRY, ELSEVIER SCIENCE INC, US, vol. 18, no. 7, 26 June 2007 (2007-06-26), pages 1232-1238, XP022131654
- WO 2006/040622

The present invention provides methods and apparatus that are particularly suited for identification of such compounds where specific uses of these advances are made. More specifically, the methods and apparatus of the present invention enable more accurate identification of such compounds.

One aspect of the invention provides a method of identifying a sample compound, the method comprising the steps of: (i) proving models of an ensemble of possible or expected structures for each of two or more known compounds; (ii) calculating a theoretical collision cross-section for each modelled structure; (iii) averaging the calculated theoretical collision cross-sections for each known compound to provide a theoretical collision cross-section value for each known compound; (iv) measuring a collision cross-section value for the sample compound using an ion mobility technique or cell; and (v) comparing the measured collision cross-section value with the calculated theoretical collision cross-section values to identify which of the two or more known compounds the sample compound most closely resembles.

The two or more known compounds may comprise two or more known isomeric or isobaric compounds, for example metabolites, e.g. hydroxylated metabolites.

The ion mobility technique or cell preferably comprises a travelling wave ion mobility technique or cell.

The method may further comprise the step of analysing a sample compound, for example a further sample compound, that may be from the same sample, e.g. using a mass spectrometer, for example to produce a mass spectrum. This analysing step may be carried out before the comparing step, e.g. in order to identify and/or further characterise the sample compound.

The step of providing models may further comprise checking simplified molecular input line entry specification strings and/or protonates / deprotonates ionisation centres, for example according to physiological pH. Additionally or alternatively, the step of providing models may comprise generating three dimensional structures and may further comprise energy minimising the three dimensional structures

Another aspect of the invention provides a computer program element, embodied on a computer readable medium comprising a computer readable program code means to execute the method steps according to the invention.

A yet further aspect of the invention provides an analytical apparatus suitable for carrying out a method as described above and/or comprising a program element as described above and/or a computer readable medium as described above, which apparatus may be specifically adapted for carrying out the method. The analytical instrument may comprise a mass spectrometer.

Embodiments of the invention will now be described by way of example only with reference to the accompanying drawings in which:

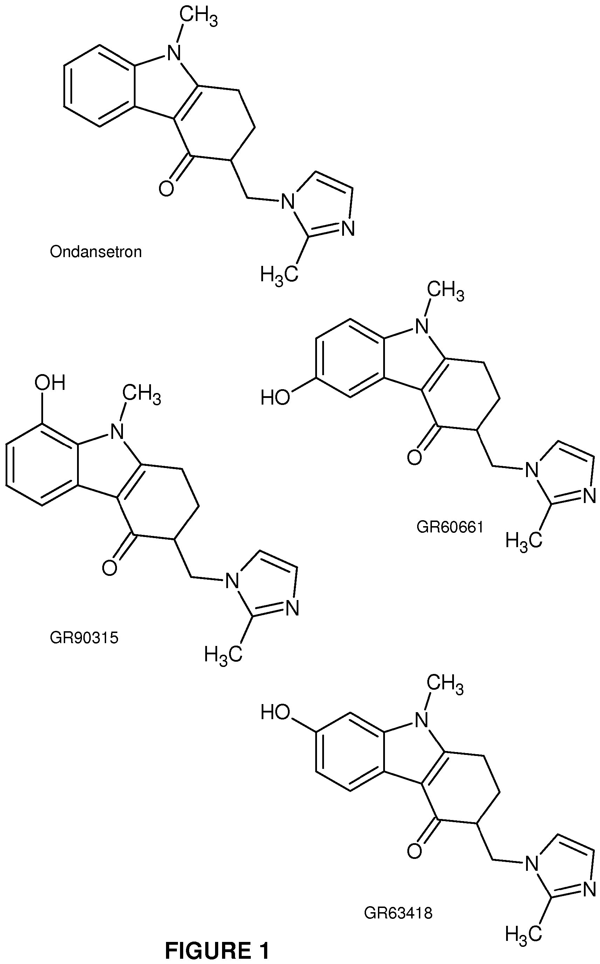

Figure 1 illustrates a parent drug ondansetron and three hydroxylated metabolites GR60661, GR90315 and GR63418;
Figure 2 illustrates the ensemble of hydroxylated metabolite GR60661 structures created by molecular dynamics and structure refinement, wherein the structures differ by degree rotation around the two rotatable bonds;

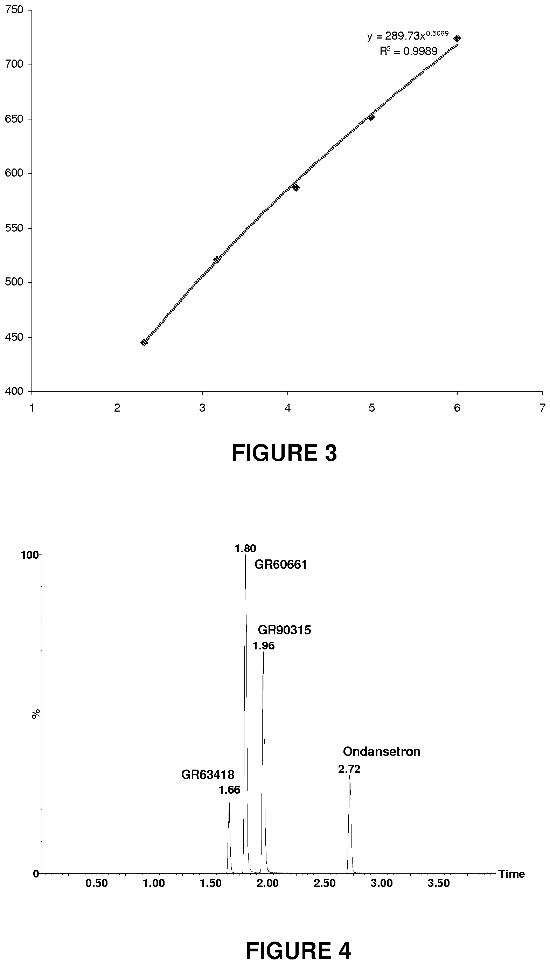

Figure 3 is a graph showing a travelling wave ion mobility calibration curve of drift-time (msec) verses adjusted collision cross-section for five polyalanine ions, wherein a power curve of the form y=ax^{b} is fitted to the data;
Figure 4 illustrates the UPLC/MS separation of ondansetron and hydroxylated metabolites GR60661, GR63418 and GR90315;
Figure 5 illustrates the collision induced dissociation MS/MS spectrum for the hydroxylated metabolite GR60661 *m*/*z* 310.1;
Figure 6 is a table showing the comparison of the collision cross-section (Ω, in Å²) values of ondansetron and hydroxylated metabolites GR60661, GR63418 and GR90315 obtained using travelling wave ion mobility measurements, Waters™ Projection Approximation (PA) and the MOBCAL™ Trajectory Method (TM) calculations;
Figure 7A is an overlay of ion mobility arrival time distributions for the parent compounds ondansteron and metabolites GR90315, GR63418 and GR60661, in order of ion mobility elution (travelling wave 1100m/sec, 3.3 mbar nitrogen);
Figure 7B is an overlay of ion mobility arrival time distributions for metabolites GR63418 and GR60661 (travelling wave 1100m/sec, 3.3 mbar nitrogen);
Figure 8 shows a DriftScope 2.1 plot of drift time (msec) verses retention time (mins) of the LC-IMS-MS analysis of ondansetron, GR60661, GR63418 and GR90315 and assigned travelling wave collision cross-section values; and
Figure 9 is a table showing the comparison of the collision cross-section (Ω, in Å²) values of methylated imidazole neutral loss fragment of ondansetron and hydroxylated metabolites GR60661, GR63418 and GR90315 obtained using travelling wave ion mobility measurements and the MOBCAL™ Trajectory Method calculations.

Experiments were performed using a hybrid quadrupole/ion mobility/orthogonal acceleration time-of-flight Synapt G2 HDMS instrument. Ion mobility separation was performed at a pressure of 3.2 mbar nitrogen with a wave velocity of 1000 m/sec and a fixed travelling wave amplitude of 40 V was used. Theoretical Ω values were calculated using MOBCAL™ and the Waters™ Ω algorithm (as described in WO2010/119289 and WO2010/11923, the contents of which are incorporated herein by reference) and compared to the travelling wave derived Ω values. Ion mobility calibration was undertaken using five singly charged polyalanine ions. Three-dimensional metabolite conformations were explored with the "Systematic Search" protocol in MOE. Ten thousand combinations of possible values for variables were explored. All conformations were minimised with the MMFF94 forcefield. The ten conformations with the lowest energy were selected for each metabolite.

The Ω value of the parent compound ondansetron and the three hydroxylated metabolites GR60661, GR63418 and GR90315 were analysed by UPLC-IMS-MS. All three hydroxylated metabolites showed different chromatographic elution time. However, the MS and MS/MS spectra are all identical, thus hampering identification. The theoretically derived Ω values for hydroxylated metabolites GR60661, GR63418 and GR90315 are 112.5 A2, 111.8 A2 and 110.9 A2 respectively. All reported values based on the Projection Approximation and Trajectory Method. Travelling wave ion mobility derived collision cross-section values are in very close agreements with the theoretically derived values, thus allowing accurate identification of the hydroxylated metabolites, even those with a collision cross-section difference of less than 1 A2. No additional structural characterization techniques, such as NMR or X-ray crystallography were required for metabolite structural determination. This is a very time efficient and effective means of identifying drug metabolites directly from biological matrices. Additionally it can be utilised in a situation where chemically synthesised standards may not be available.

The invention provides a new methodology which combines molecular modelling, theoretical Ω calculations and travelling wave ion mobility derived Ω measurements, to successfully indentify a number of drug metabolites, which cannot be differentiated by traditional MS/MS techniques. *In-silico* molecular modelling is used to create an ensemble (approximately 10,000) of possible metabolite structures (in this case study hydroxylated) from the parent compound ondansetron. The theoretical Ω values were calculated for the ten lowest energy structures. The theoretical Ω values are averaged for each structure (metabolite) therefore, providing an average of all the possible structures/conformations the molecule may "explore" in the gas-phase. The travelling wave Ω values are then derived for each metabolite by LC-IMS-MS.

### Instrumentation

Synapt G2 HDMS was operated in ion mobility mode (positive ion mode). The travelling wave ion mobility cell was operated at a pressure of 3.3 mbar nitrogen with a travelling wave speed of 1000 m/sec and a pulse amplitude of 40 V.

Acquity UPLC was operated at a flow rate of 600uL/min. Solvent A was 5 mM ammonium acetate. Solvent B was 100% acetonitrile. Chromatographic separation was performed on a 2.1 x 50 mm BEH C18 1.7 um column utilising a 5 minute gradient, starting at 5% solvent B, increasing to 30% solvent B, over 3 minutes.

### Molecular Modelling

Simplified Molecular Input Line Entry Specification (SMILES) strings for all the compounds were transformed into 3D structures utilizing the software package Molecular Operating Environment (MOE). The following steps were performed: First, wash smiles. This checks the smiles string and protonates/deprotonates ionisation centers according to physiological pH. Second, generate 3-D structures (converts 2-D into 3-D). Third, energy minimise the 3-D structures. Once the 3D compounds were generated, the conformations were explored with the "Systematic Search" protocol in MOE. This is suitable since ondansetron only has two rotatable bonds (3 if you count the -OH in the metabolites) so, the combinatorial explosion is controlled.

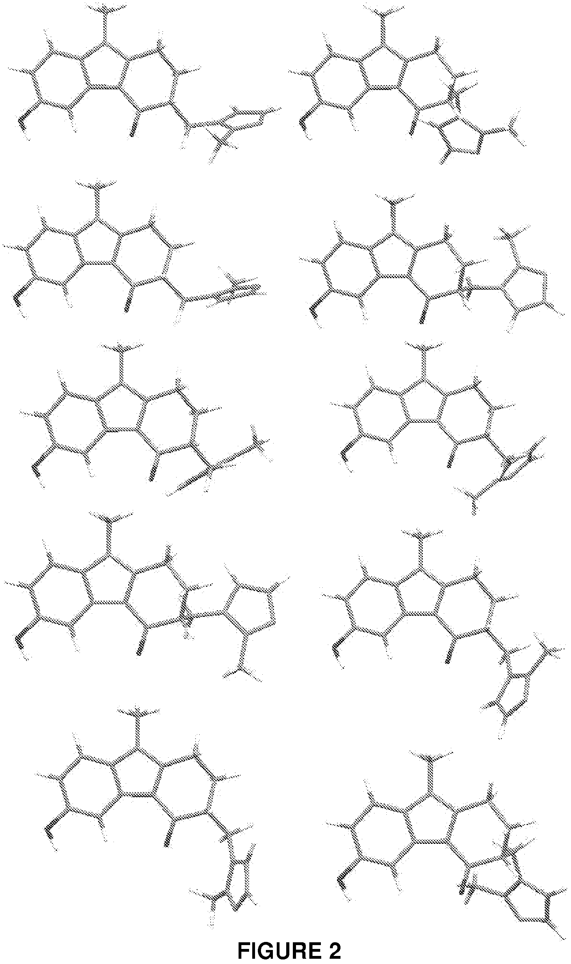

Possible conformations for the cyclohexane ring were also considered. In total, around 10,000 combinations of possible values for the variables explored were generated. All the conformations were energy minimised with the MMFF94 forcefield and the ten conformations (Figure 2) with the lowest energy were selected for each metabolite.

Energy minimized candidate structures were also created for the fragment ions relating to the neutral loss of the methylated imidazole structure *(m*/*z* 228.1, C₁₄O₂NH₁₃, for hydroxylated metabolites GR60661, GR90315, GR63418 and *m*/*z* 212.1, C₁₄ONH₁₃, for ondansetron). The basis set HF/6-31G++ was used for structure refinement.

### Theoretical Collision Cross-section Calculations

Theoretical Ω values were calculated using the open source software program MOBCAL™. Briefly, the MOBCAL™ output is based on three different models/algorithms which calculate the theoretical Ω of any molecule possessing a 3-dimensional coordinate file. The models/algorithms are the Projection Approximation (PA), Exact Hard Sphere Scattering (EHSS) and the Trajectory Method (TM), however, only the MOBCAL™ Trajectory Method Ω value will be reported. The Waters™ Ω algorithm, based in DriftScope 2.1 was also utilized. The values obtained from the MOBCAL™ and the Waters™ Ω algorithm were then compared to the travelling wave derived Ω values.

### Travelling Wave Ion Mobility Calibration

Travelling wave ion mobility calibration of the Synapt G2 HDMS instrument was undertaken using five singly charged polyalanine ions (AAA, AAAA, AAAAA, AAAAAA and AAAAAAA) whose absolute Ω values are known from standard drift-tube studies. The polyalanine ions and their respective absolute Ωs used for T-Wave ion mobility calibration were as follows: - AAA *(m*/*z* 232.1), 89.0 Å², AAAA *(m*/*z* 303.1), 102.9 Å², AAAAA *(m*/*z* 374.1) 115.0 Å², AAAAAA *(m*/*z* 445.2) 127.0 Å² and AAAAAAA *(m*/*z* 516.2) 140.5 Å².

For the travelling wave calibration, the drift tube Ω values were corrected by multiplying by the square root of the reduced mass and dividing by the charge state of the calibrant species to provide a term related directly to 1/K. The measured travelling wave drift times were finally corrected for their mass-independent and mass-dependent flight times between the travelling wave ion mobility cell and the ToF analyser. The adjusted Ω values were plotted against corrected drift time (msec) values determined using the T-wave device and the data fitted using an empirically determined power-law form, y = **a**x^{b} (Figure x). The derived coefficients of a and b obtained from the calibration curve were used to calculate the Ω value of ondansetron and related hydroxylated metabolites from their measured travelling wave drift times obtained under identical operating conditions to those of the calibrant species polyalanine.

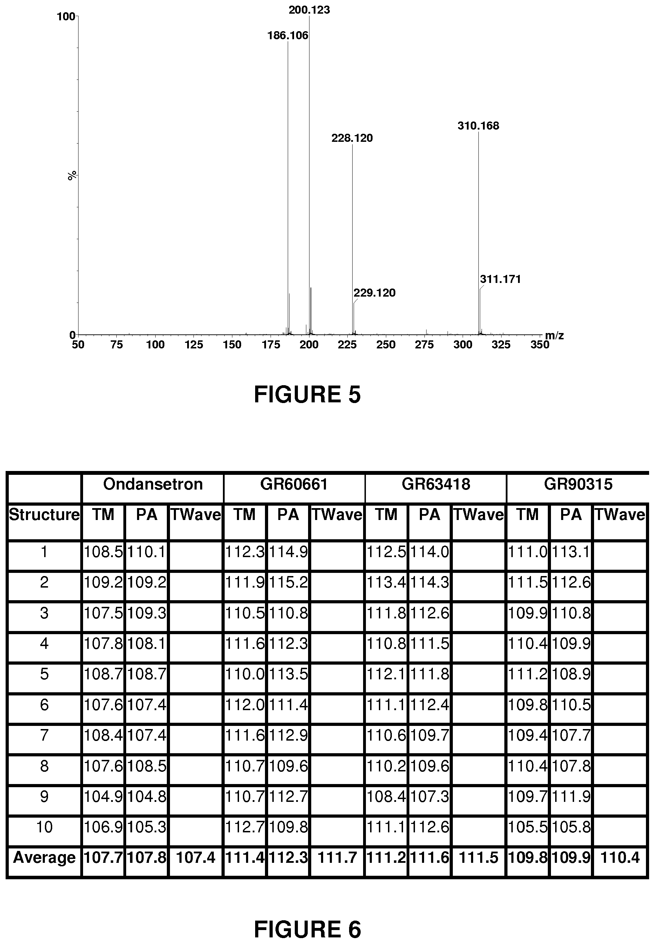

It was found that the three hydroxylated metabolites can all be separated by UPLC. However, in the absence of any chemical standard as a reference, and one was analysing metabolites directly from a biological matrix, it would be very challenging to identify each metabolite based on the MS or the MS/MS spectra alone. Figure 5 shows the MS/MS spectra of hydroxylated metabolite GR60661. The fragmentation spectra for metabolites GR63418 and GR90315 are identical to that presented in Figure 5. Therefore, one cannot identify the hydroxylated metabolites based purely on their MS/MS fragmentation pattern.

The present invention provides a new approach to metabolite structural elucidation and characterisation by generating an ensemble of energy minimised metabolite structures *in-silico* as described above in the Molecular Modelling section. This allows for the theoretical Ω calculation of the metabolite structures, which can then be compared to the travelling wave ion mobility measured Ω value, based on the LC-IMS-MS experiment. Figure 6 shows the comparison of the MOBCAL™ Trajectory Method, the Waters™ PA algorithm derived Ω values for the ten lowest energy structures of ondansetron, GR60661, GR63418 and GR90315 and a comparison to the travelling wave derived Ω values.
Observed in Figure 6 is the substantial agreement of the theoretically derived Ω values and the travelling wave derived Ω values. The trend of the theoretical and travelling wave derived Ω values also match, in that metabolite GR60661 possesses the largest Ω value and GR90315, the smallest Ω value.

This demonstrates that it is possible with the methods of the present invention to generate accurate theoretical Ω values for metabolites, compare them to gas-phase Ω measurements and use this comparison to assign and identify the position of the hydroxylation site within the metabolite. In the example shown herein, structures GR60661 and GR63418 only differ in theoretical Ω value of 0.2 Å² (111.4 and 111.2 respectively, based on the PA method).

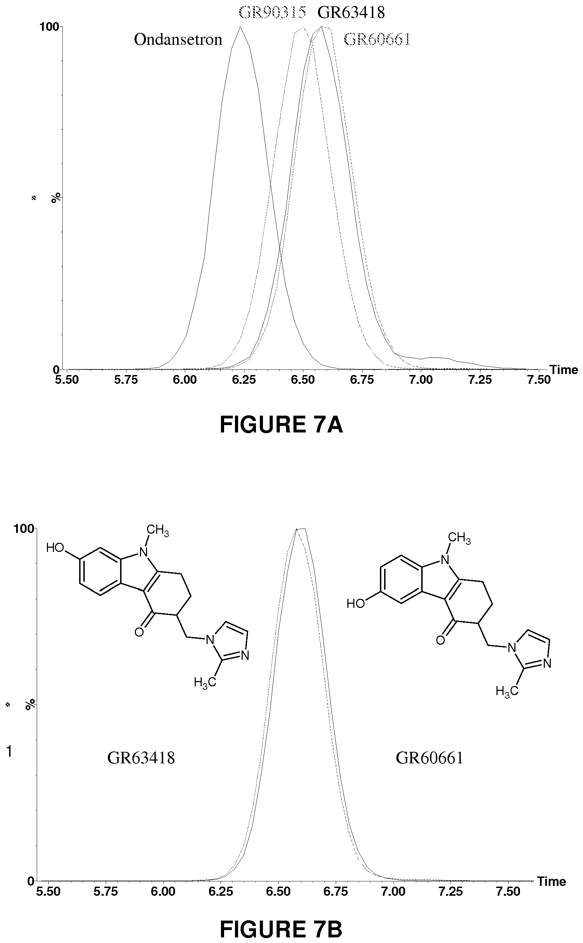

This small difference in theoretical collision cross-section can be measured and characterised using the Synapt G2 instrument. This is demonstrated in Figure 7A & 7B where the arrival time distributions of all metabolites have been overlaid. The small arrival time distribution difference of metabolites GR60661 and GR63418 is reproducible and measureable (Figure 7B). Additionally, the addition of the hydroxyl moiety results in a measurable increase in collision cross-section of 3-4 Å², depending on which metabolite is being considered.

### IMS-MS of fragment ions

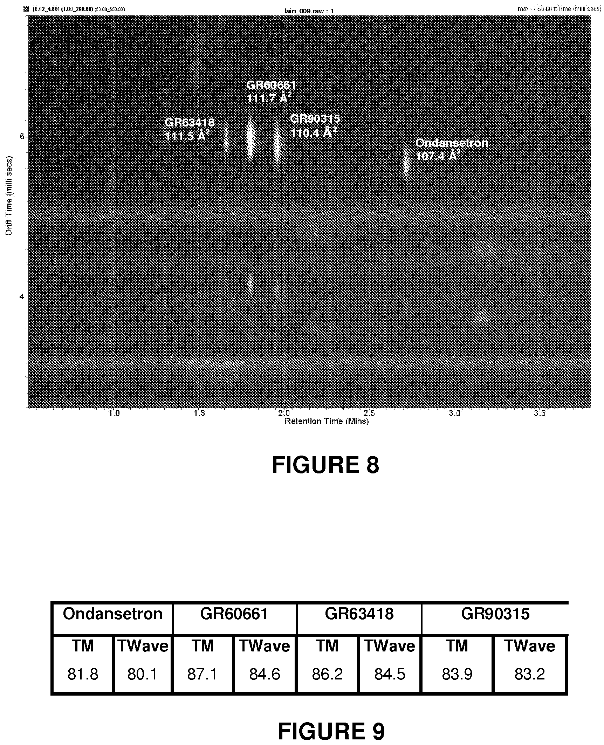

Potential candidate structures can also be created and optimised for fragment ions. The metabolite fragment ion m/z 228.1, corresponding to the methylated imidazole neutral loss species was selected due to the absence of any rotatable bonds and almost planar structure. Considering the two closest structurally related metabolites, in terms of Ω value, GR60661 and GR63418, it is also possible to differentiate them based on the fragment ion *(m*/*z* 228.1) conformation, both T-wave and theoretically derived. By first considering the T-wave derived Ω values, then the arrival time distributions are reproducibly offset by a value of 100usec (equating to a 0.1 Å² difference) with GR63418 always preceding GR60661 (Figure 9). The trend of the MOBCAL™ trajectory method derived Ω values follows that of the instrument derived values, albeit, a larger difference of 1.0 Å² between GR63418 and GR60661.

Based on this limited sample set, it would be challenging to predict the position of the hydroxyl moiety of the meta (GR63418) and para-hydroxylated (GR60661) metabolites by comparing theoretical and T-wave derived Ω values. The situation is more clear when considering the ortho-hydroxylated metabolite (GR90315). The T-wave and theoretically derived values correlate very well (Figure 9).

### Using carbon dioxide as the ion mobility drift gas

As demonstrated previously by Hill (Anal Chem 2000) the ATDs of compounds under investigation can change dramatically depending on which drift gases are used. Herein, it is demonstrated that using the more polarizable carbon dioxide as the drift gas, the metabolite GR63418 has a longer drift time than GR60661. This is opposite to what is observed when using the less polar nitrogen as the drift gas.

### Benefits

The technique of the present invention is rapid, since it does not require lengthy chemical synthesis pathways for production of metabolites standards. Utilising LC-IMS-MS, it is possible to determine the Ω values of the metabolites directly from biological matrices. Additionally, and importantly, this method does not require skilled interpretation of NMR spectra to derive metabolite structures. Figure 8 shows the LC-IMS-MS data, with annotated travelling wave, displayed in DriftScope 2.1, as a function of drift time verses chromatographic retention time.

The invention allows travelling wave ion mobility combined with *in-silico* molecular modelling of hydroxylated metabolite structures can be successfully implemented to positively identify the hydroxylated metabolites based on their collisional cross-sections. This technique can be rapidly utilised to supplement all traditional methodologies of metabolite identification, such as MS/MS.

This IM-MS based approach can enable a researcher to identify metabolites without the need for metabolite synthesis and/or NMR structural elucidation. In cases where this method can not identify all metabolites present, since some metabolites will possess an identical collision cross-sections (both theoretically and instrument ion mobility derived), this method can be used to "rule-out" potential structures on the basis of their theoretical Ω:T-Wave derived Ω comparison.

It will be appreciated by those skilled in the art that any number of combinations of the aforementioned features and/or those shown in the appended drawings provide clear advantages over the prior art and are therefore within the scope of the invention described herein.

## Claims

1. A method of identifying a sample compound, the method comprising the steps of:
i. providing models of an ensemble of possible structures for each of two or more known compounds;
ii. calculating a theoretical collision cross-section for each modelled structure;
iii. averaging the calculated theoretical collision cross-sections for each known compound to provide a theoretical collision cross-section value for each known compound;
iv. measuring a collision cross-section value for the sample compound using an ion mobility technique; and
v. comparing the measured collision cross-section value with the theoretical collision cross-section values to identify which of the two or more known compounds the sample compound most closely resembles.

2. Method according to claim 1 wherein the two or more known compounds comprise two or more known isomeric or isobaric compounds.

3. Method according to any preceding claim, wherein the ion mobility technique comprises a travelling wave ion mobility technique.

4. Method according to any preceding claim further comprising the step of analysing a further sample compound from the same sample using a mass spectrometer to produce a mass spectrum before the comparing step in order to identify or further characterise the sample compound.

5. Method according to any preceding claim, wherein the step of providing models further comprises checking simplified molecular input line entry specification strings and protonates / deprotonates ionisation centres according to physiological pH.

6. Method according to any preceding claim, wherein the step of providing models further comprises generating three dimensional structures; optionally, wherein the step of providing models further comprises energy minimising the three dimensional structures.

7. Method according to any preceding claim, wherein the isomeric or isobaric compound structures comprise hydroxylated metabolites.

8. A computer program element embodied on a computer readable medium comprising computer readable program code means for causing a processor to execute a procedure to implement the method of any one of claims 1 to 7.

9. An analytical apparatus adapted to carry out a method according to any one of claims 1 to 7 or comprising a program element according to claim 8.

## Patentansprüche

1. Verfahren zur Identifikation einer Probenverbindung, wobei das Verfahren die folgenden Schritte umfasst:
i. Bereitstellen von Modellen eines Ensembles von möglichen Strukturen für jede von zwei oder mehr bekannten Verbindungen;
ii. Berechnen eines theoretischen Kollisionsquerschnitts für jede modellierte Struktur;
iii. Mitteln der berechneten theoretischen Kollisionsquerschnitte für jede bekannte Verbindung, um einen theoretischen Kollisionsquerschnittswert für jede bekannte Verbindung bereitzustellen;
iv. Messen eines Kollisionsquerschnittswerts für die Probenverbindung unter Verwendung einer lonenmobilitätstechnik; und
v. Vergleichen des gemessenen Kollisionsquerschnittswerts mit den theoretischen Kollisionsquerschnittswerten, um zu identifizieren, welcher der zwei oder mehr bekannten Verbindungen die Probenverbindung am ähnlichsten ist.

2. Verfahren nach Anspruch 1, worin die zwei oder mehr bekannten Verbindungen zwei oder mehr bekannte isomere oder isobare Verbindungen umfassen.

3. Verfahren nach einem vorhergehenden Anspruch, worin die lonenmobilitätstechnik eine Wanderwellen-Ionenmobilitätstechnik umfasst.

4. Verfahren nach einem vorhergehenden Anspruch, ferner umfassend den Schritt des Analysierens einer weiteren Probenverbindung aus derselben Probe unter Verwendung eines Massenspektrometers zur Erzeugung eines Massenspektrums vor dem Vergleichsschritt, um die Probenverbindung zu identifizieren oder weiter zu charakterisieren.

5. Verfahren nach einem vorhergehenden Anspruch, worin der Schritt des Bereitstellens von Modellen ferner die Überprüfung von SMILES- (Simplified Molecular Input Line Entry Specification) Strings umfasst und lonisierungszentren gemäß dem physiologischen pH protoniert/deprotoniert.

6. Verfahren nach einem vorhergehenden Anspruch, worin der Schritt des Bereitstellens von Modellen ferner die Erzeugung von dreidimensionalen Strukturen umfasst; wahlweise, worin der Schritt des Bereitstellens von Modellen ferner die Energieminimierung der dreidimensionalen Strukturen umfasst.

7. Verfahren nach einem vorhergehenden Anspruch, worin die isomeren oder isobaren Verbindungsstrukturen hydroxylierte Metabolite umfassen.

8. Computerprogrammelement, das auf einem computerlesbaren Medium realisiert ist, umfassend computerlesbare Programmcodemittel zum Bewirken dessen, dass ein Prozessor eine Prozedur zum Implementieren des Verfahrens nach einem der Ansprüche 1 bis 7 ausführt.

9. Analytische Vorrichtung, die zum Durchführen eines Verfahrens nach einem der Ansprüche 1 bis 7 ausgelegt ist oder ein Programmelement nach Anspruch 8 umfasst.

## Revendications

1. Procédé d'identification d'un composé d'échantillon, le procédé comprenant les étapes consistant à :
(i) fournir des modèles d'un ensemble de possibles structures pour chacun d'au moins deux composés connus ;
(ii) calculer une section transversale de collision théorique pour chaque structure modélisée ;
(iii) calculer la moyenne des sections transversales de collision théoriques calculées pour chaque composé connu afin de fournir une valeur de section transversale de collision théorique pour chaque composé connu ;
(iv) mesurer une valeur de section transversale de collision pour le composé d'échantillon au moyen d'une technique de mobilité ionique ; et
(v) comparer la valeur de section transversale de collision mesurée aux valeurs de section transversale de collision théoriques afin d'identifier à quels au moins deux composés connus le composé d'échantillon ressemble le plus.

2. Procédé selon la revendication 1, dans lequel les au moins deux composés connus comprennent au moins deux composés isomères ou isobares connus.

3. Procédé selon l'une quelconque des revendications précédentes, dans lequel a technique de mobilité ionique comprend une technique de mobilité ionique à ondes progressives.

4. Procédé selon l'une quelconque des revendications précédentes, comprenant en outre l'étape consistant à analyser un autre composé d'échantillon provenant du même échantillon au moyen d'un spectromètre de masse pour produire un spectre de masse avant l'étape de comparaison, afin d'identifier ou de caractériser davantage le composé d'échantillon.

5. Procédé selon l'une quelconque des revendications précédentes, dans lequel l'étape consistant à fournir des modèles consiste en outre à vérifier des chaînes SMILES (Simplified Molecular Input Line Entry Spécification) et des centres d'ionisation par protonation/déprotonation selon un pH physiologique.

6. Procédé selon l'une quelconque des revendications précédentes, dans lequel l'étape consistant à fournir des modèles consiste à générer des structures tridimensionnelles ; éventuellement, dans lequel l'étape consistant à fournir des modèles consiste en outre à minimiser énergétiquement les structures tridimensionnelles.

7. Procédé selon l'une quelconque des revendications précédentes, dans lequel les structures de composés isomères ou isobares comprennent des métabolites hydroxylés.

8. Élément de programme informatique intégré sur un support utilisable par ordinateur, comprenant un moyen de code de programme lisible par ordinateur amenant un processeur à exécuter une procédure pour mettre en oeuvre le procédé selon l'une quelconque des revendications 1 à 7.

9. Appareil analytique conçu pour exécuter un procédé selon l'une quelconque des revendications 1 à 7 ou comprenant un élément de programme selon la revendication 8.
